Europäisches Patentamt

European Patent Office

Office européen des brevets

(19)

(11) **EP 0 799 600 B1**

(12) ## EUROPEAN PATENT SPECIFICATION

(45) Date of publication and mention
of the grant of the patent:
**08.09.2004   Bulletin 2004/37**

(51) Int Cl.[7]: **A61B 6/00**, G01N 23/04,
G03B 42/02

(21) Application number: **97105163.6**

(22) Date of filing: **26.03.1997**

(54) **Phase-contrast X-ray imaging system**

Bilderzeugungssystem mit Phasenkontrast für Röntgenstrahlen

Imagerie à contraste de phase pour rayons X

(84) Designated Contracting States:
**DE FR NL**

(30) Priority: **29.03.1996   JP 7585996**

(43) Date of publication of application:
**08.10.1997   Bulletin 1997/41**

(73) Proprietor: **Hitachi, Ltd.**
**Chiyoda-ku, Tokyo 101 (JP)**

(72) Inventor: **Momose, Atsushi**
**Hiki-Gun, Saitama-Ken (JP)**

(74) Representative: **Strehl Schübel-Hopf & Partner**
**Maximilianstrasse 54**
**80538 München (DE)**

(56) References cited:
WO-A-95/05725          US-A- 5 016 267
US-A- 5 173 928          US-A- 5 259 013
US-A- 5 319 694

• **BECKER; BONSE: 'The skew-symmetric
two-crystal X-ray interferometer'
J.APPL.CRYST. vol. 7, 1974, COPENHAGEN,
pages 593 - 598**

## Description

BACKGROUND OF THE INVENTION

Field of the Invention

The present invention relates to a phase-contrast X-ray imaging system, in detail relates to an X-ray imaging system utilizing a fact that the sensitivity of an image provided by a phase-contrast X-ray imaging system is extremely high, compared with that provided by a conventional type X-ray imaging method depending upon absorption contrast. The present invention is suitable for observing biological soft tissues and others the X-ray absorbing power of which is small and as a relatively wide visual field for observation can be secured, the present invention can be used for a medical diagnosis apparatus.

Description of the Related Art

All currently realized X-ray imaging systems obtain an image contrast based upon the quantity of absorbed X-rays. The heavier an element is, the more X-rays are absorbed and the more heavy elements are included in a subject, the more easily the shadow of an X-ray can be formed. In the meantime, a substance made of light elements which does not absorb X-rays so much is too transparent for X-rays and a sufficient contrast cannot be obtained. For a medical X-ray diagnosis apparatus, a method of emphasizing the image contrast of such soft tissues which are difficult to observe by injecting contrast media including heavy elements into a subject is adopted if the method is allowed. In case there are no suitable contrast media for an X-ray diagnostic apparatus (mammography) for diagnosing a mammary cancer, efforts to enhance the sensitivity of an image even if a little using X-rays the energy of which is relatively low are made. This is because X-ray absorption is in inverse proportion to the third power of X-ray energy and a contrast is readily formed. However, as the dose of X-rays is also in inverse proportion to the third power of X-ray energy, the increase of the dose of X-rays caused by using X-rays the energy of which is low must be submitted. It cannot be said that an image enough to diagnose using can be obtained.

In the meantime, there is an imaging method of obtaining a contrast by phase shift instead of X-ray absorption. As for light elements, the phase interaction cross section of X-ray phase shift is approximately a thousand times as large as the interaction cross section of X-ray absorption, observation which is a few hundred times as sensitive as prior observation is enabled. This shows that a subject the shadow by X-rays of which is difficult to form by a conventional type method can be observed without using special contrast media and is proved in an experiment. An X-ray phase-contrast image is observed using an X-ray interferometer, however, as the size of an interferometer which can be manufactured is limited, its observational field is narrow and application as it is to a medical diagnosis apparatus is difficult. For an example of the visual field of a few millimeters, Phase-contrast X-ray radiography (A. Momose, et al., Med. Phys., 22, 375-380 (1995)) and Phase-contrast computed tomography (A. Momose, et al., Rev. Sci. Instrum. 66, 1434-1436 (1995), the United States patent application 5, 173, 928) can be given.

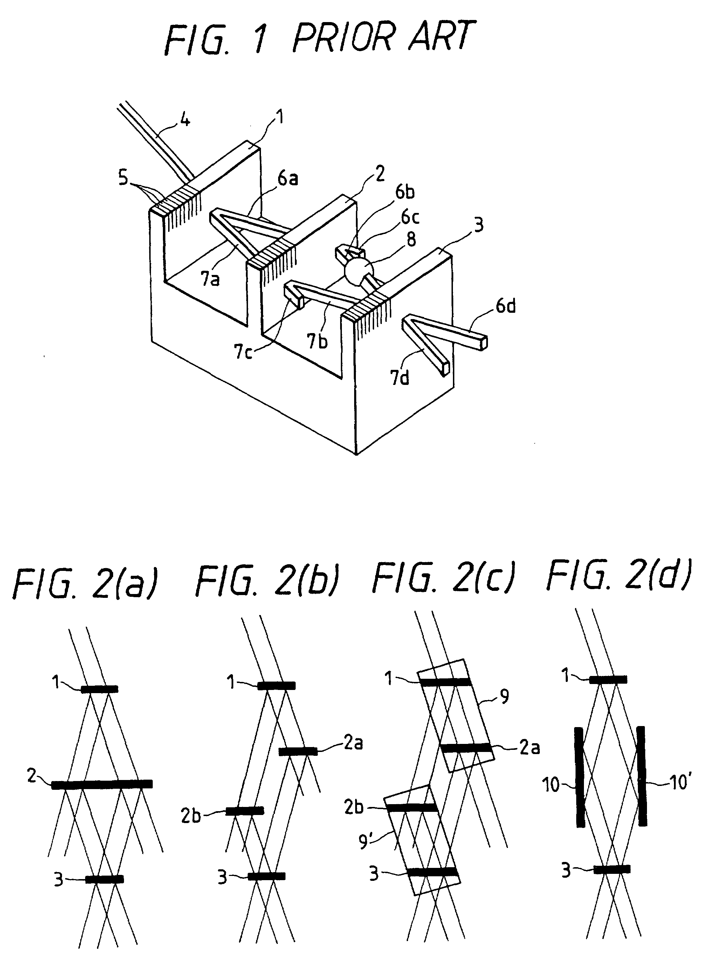

Currently known X-ray interferometers are produced by integrally carving the body from a single crystal ingot made of silicon and others as shown in Fig. 1. Three crystalline plates 1 to 3 are arranged in parallel and at an equal interval one another, when an incident X-ray 4 meets the diffraction condition of a lattice plane 5, the incident X-ray 4 is separated into two beams 6a and 7a, each beam is similarly separated into two beams 6b and 6c and into two beams 7b and 7c again by a second crystalline plate 2, the beams 6b and 7b are connected by a third crystalline plate 3 and interfere each other. That is, the three crystalline plates 1 to 3 function as X-ray half mirrors. When a subject 8 is inserted in the path of one beam, for example the beam 6b, the phase of the X-ray is shifted and an interference pattern is formed by X-ray beams 6d and 7d through a third crystalline plate 3. As the size of an observation field is equivalent to the thickness of an X-ray beam through an interferometer and two beams formed in an interferometer are required to be separated completely, that is, to be not overlapped, the whole interferometer is required to be enlarged to thicken a beam. When it is considered that the whole interferometer is carved from a single crystal ingot and the size of a currently available silicon ingot is limited, a securable observation field is at the largest approximately 2 square centimeters.

An X-ray interferometer in which respective two X-ray half mirrors are formed in two independent crystalline units is reported on pages 593 to 598 written by P. Becker and U. Bonse in vol. 7 of J. Appl. Cryst. published in 1974. This paper discloses a phase contrast X-ray imaging system in accordance with the first part of claim 1.

SUMMARY OF THE INVENTION

it is an object of the present invention to provide a phase contrast X-ray imaging system which is suited for phase-contrast mammography, phase-contrast angiography and phase-contrast X-ray computed tomography.

This object is met by the invention characterised in claim 1.

BRIEF DESCRIPTION OF THE DRAWINGS

   Fig. 1 shows a well-known integrated X-ray interferometer;

Figs. 2 (a) to (d) show the basic beam path of an X-ray interferometer constituted by an independent X-ray half mirror;

Figs. 3 (a) to (c) show the difference in the type of diffraction caused by the difference in an angle a formed between a crystal surface and a lattice plane;

Fig. 4 shows the shape of an X-ray half mirror unit used in a first embodiment according to the present invention and its adjustment axes;

Fig. 5 shows a state in which the X-ray half mirror unit shown in Fig. 4 is carved from a cylindrical silicon ingot;

Fig. 6 is a plan and a side view showing an example of the constitution of a piezoelectric element driving stage for a rotation axis θ in the first embodiment according to the present invention;

Fig. 7 is a plan and a side view showing an example of the constitution of a piezoelectric element driving stage for a rotation axis ω in the first embodiment according to the present invention;

Figs. 8 (a) to (d) show the constitution of a phase plate for a fringe scanning method;

Fig. 9 is a plan showing the whole constitution of a phase-contrast mammographic system equivalent to the first embodiment according to the present invention;

Fig. 10 is a perspective drawing showing a state in which a chamber is provided to the phase-contrast mammographic system equivalent to the first embodiment according to the present invention;

Fig. 11 is a perspective drawing showing the outline of the inside structure of the chamber for which an oscillation preventing measure is taken of the phase-contrast mammographic system equivalent to the first embodiment according to the present invention;

Fig. 12 shows the shape of an X-ray half mirror used in a second embodiment according to the present invention and its adjustment axes;

Fig. 13 shows a state in which the X-ray half mirror shown in Fig. 12 is carved from a cylindrical silicon ingot;

Fig. 14 is a plan showing an example of the constitution of a piezoelectric element driving stage for a parallel displacement axis x in the second embodiment according to the present invention;

Fig. 15 shows the whole constitution of a phase-contrast mammographic system equivalent to the second embodiment according to the present invention;

Fig. 16 is a block diagram for driving and controlling the phase-contrast mammographic system; and

Fig. 17 shows an example of the constitution of an X-rays source as a linear light source and the positional relationship between the X-rays source and an interferometer.

## DESCRIPTION OF THE PREFERRED EMBODIMENTS

Figs. 2 show some basic constitutional examples of the beam path of an X-ray interferometer constituted by an independent X-ray half mirror. Fig. 2 (a) shows an example in which the half mirrors 1 to 3 shown in Fig. 1 are simply independently separated, Fig. 2 (b) shows an example in which the central half mirror is further separated into two independent half mirrors 2a and 2b and space for inserting a subject (an interval between the half mirror 2a and the half mirror 3) is expanded by shifting them in the reverse direction and Fig. 2 (c) shows an example in which the two half mirrors 1 and 2a and the two half mirrors 2b and 3 respectively in the example shown in Fig. 2 (b) are respectively integrated as one group and as a whole, two independent sets of half mirrors are constituted by units 9 and 9'. When this example is viewed from only the shape of the arranged half mirrors, it is related to research by P. Becker et al. Further, Fig. 2 (d) shows an example in which X-ray reflecting mirrors 10 and 10' are used in place of the central half mirror 2. As the central half mirrors 2, 2a and 2b shown in Figs. 2 (a) to (c) only function as a mirror for actually changing the propagation direction of X-rays, the reflecting mirrors are used as shown in Fig. 2 (d) so that the intensity of a beam is prevented from being lost by a half mirror.

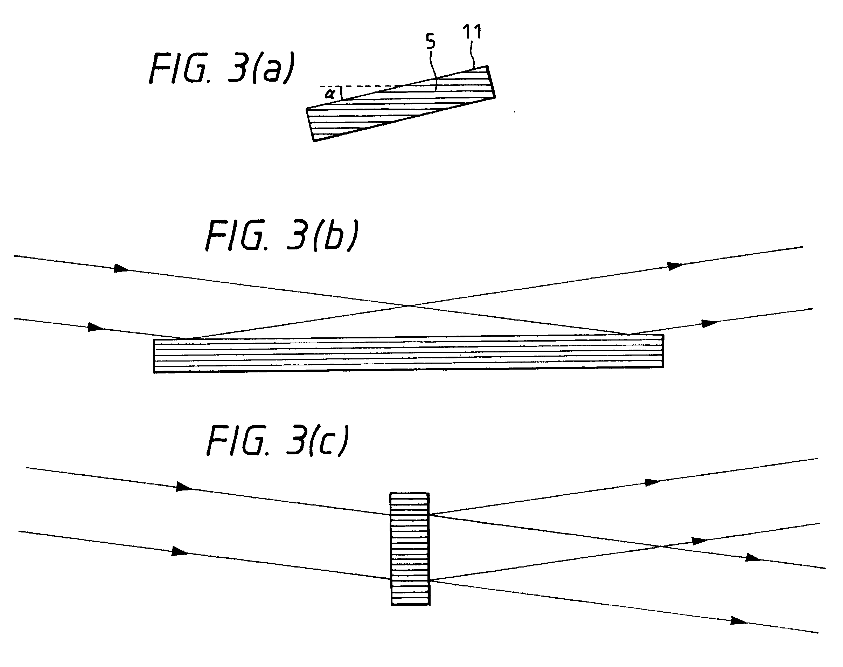

Figs. 3 (a) to (c) are schematic drawings showing the difference in the type of diffraction depending upon the difference in an angle $\alpha$ between a crystal surface and a lattice plane and show the difference between the X-ray reflecting mirror and the X-ray half mirror due to a crystal. Any drawing shows the section of a crystal and the lattice plane of a crystal is shown by a transverse line in drawings. If an angle between a crystal surface 11 and a lattice plane 5 related to diffraction is '$\alpha$' as shown in Fig. 3 (a), the mirror functions as an X-ray reflecting mirror as shown in Fig. 3 (b) ($\alpha = 0°$ in the case) if Bragg diffraction angle is θB and $\alpha < \theta B$. If $\alpha > \theta B$, the mirror functions as an X-ray half mirror as shown in Fig. 3 (c) ($\alpha = 90°$ in that case). A full line including an arrow shows an incident or outgoing X-ray in these drawings. In the case of Fig. 3 (b), an X-ray which meets Bragg diffraction condition is reflected at the reflection factor of 80 to 90% and efficiency as a mirror is excellent, compared with that in the case of Fig. 3 (c), however, there is inconvenience that a long reflecting surface is required to widen a beam as in the present invention.

The constitution of an interferometer may be another, however, it should be considered so that the length of two beam paths is substantially equal. This is because the coherence of an X-ray beam is generally not complete, the larger the difference in the length of paths is, the more coherence is deteriorated and the more the visibility of an observed interference pattern is deteriorated.

In the case of any constitution shown in Figs.

2, the relative position between each X-ray half mirror or between each X-ray reflecting mirror is required to be adjusted at finer precision than the wavelength of an X-ray. A mechanism for adjusting an angle so that Bragg diffraction condition is met is also required.

First Embodiment

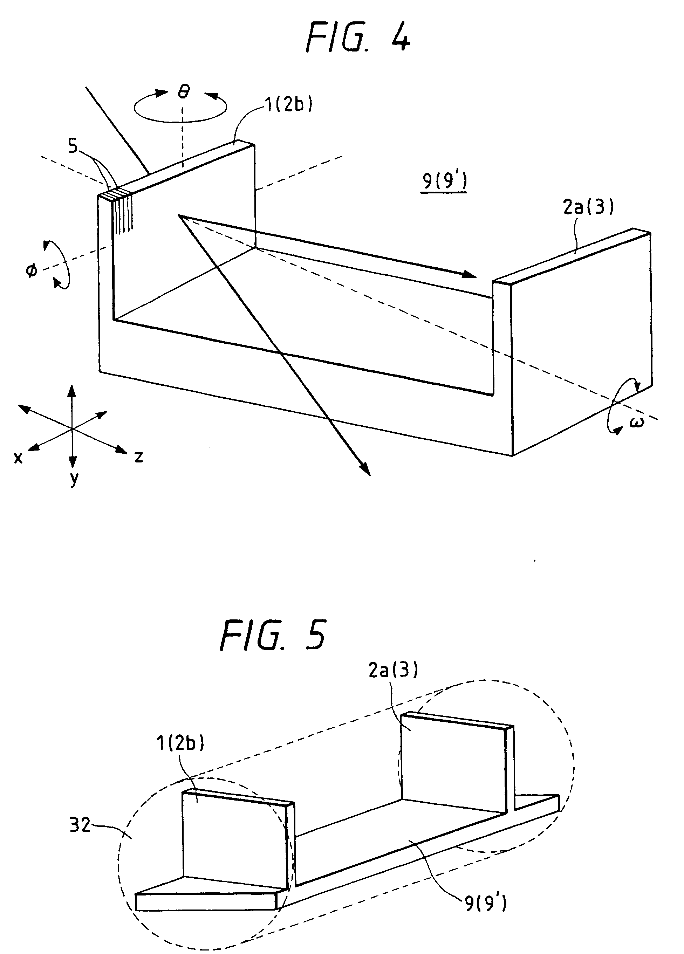

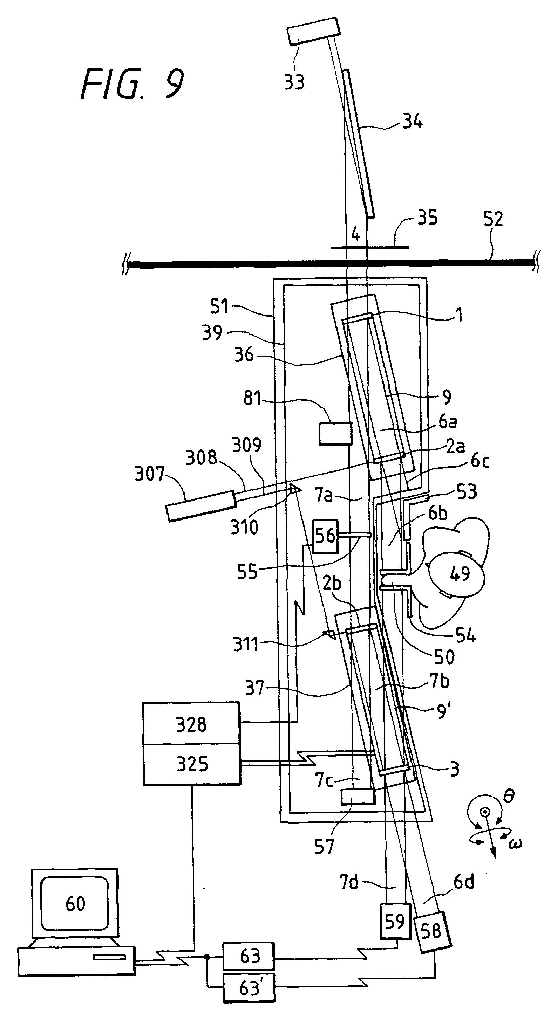

Fig. 4 shows the shape of an X-ray half mirror unit and its adjustment axes equivalent to a first embodiment according to the present invention of a phase-contrast mammographic system consisting of the units 9 and 9' provided on a common board constituted by the respective sets of the two X-ray half mirrors 1 and 2a and the two X-ray half mirrors 2b and 3 respectively shown in Fig. 2 (c). A thick full line including arrows in Fig. 4 shows an X-ray which is incident on the center of the half mirror and outgoing from the center. A reference number 5 representatively denotes a part of the lattice plane of a crystal, the direction of a normal on the lattice plane 5 is x-axis, the direction of a normal on a scattering plane (a plane including an arrow showing the propagation direction of an X-ray beam in the drawing) is y-axis and an axis perpendicular to x-axis and y-axis is z-axis. Rotation axes around x-, y- and z-axes are respectively $\phi$-axis, $\theta$-axis and $\omega$-axis. If a unit provided with this constitution is used, there is an advantage that parallel movement on x-, y- and z-axes has no effect upon coherence and adjustment is not required. This is because as the two X-ray half mirrors are simultaneously moved in parallel, an effect upon the phase of an X-ray is canceled out. For rotation on $\theta$-axis, reflection (440) is used and if an interval between the X-ray half mirrors of the unit is 80 cm, precision not more than $1 \times 10^{-10}$ rad. is required. For rotation on $\omega$-axis, reflection (440) is used and if an interval between the X-ray half mirrors of the unit is 80 cm, the wavelength of an X-ray is 0.2 Å and distance between an X-ray source and the imaging apparatus (distance between an X-ray source 33 and an X-ray two-dimensional sensor 59 in an embodiment shown in Fig. 9) is 10 m, precision not more than $1 \times 10^{-7}$ rad. is required. For $\phi$-axis, particularly severe fine adjustment is not required. Therefore, only rotation on $\omega$-axis and $\theta$-axis require adjustment.

Fig. 5 shows a state in which the respective sets of the two X-ray half mirrors 1 and 2a and the two X-ray half mirrors 2b and 3 as the units 9 and 9' provided on a common board are carved from one ingot 32. As shown in Fig. 5, if the effective area of the X-ray half mirror is 10 square cm in case FZ silicon with the diameter of nominal six inches is used, it is easy to obtain the interval of approximately 80 cm between the X-ray half mirrors. As a beam is required to be propagated substantially along the respective longitudinal directions of the units 9 and 9' respectively consisting of the X-ray half mirrors as shown in Fig. 2 (c) if the units 9 and 9' are carved from a cylindrical silicon monocrystalline ingot, a limitation is imposed on the growth axis of the ingot and the lattice plane related to diffraction. For example, when an ingot grown in the direction tilted from <111> axis to <110> axis by six degrees is used in case an X-ray of 60 keV is used for reflection (440), a wide visual field can be efficiently secured.

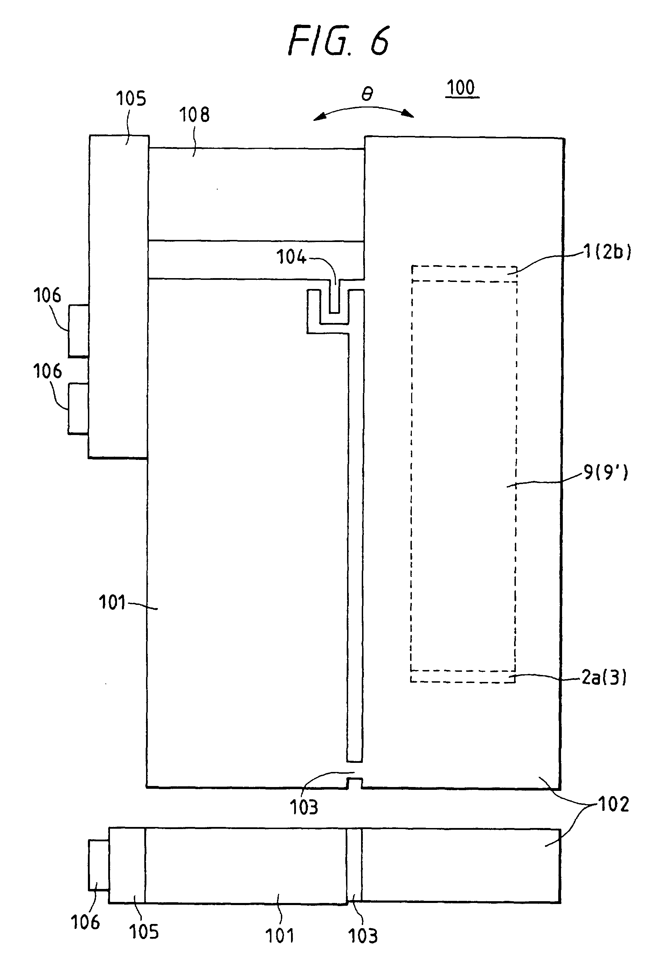

Fig. 6 is a plan and a side view showing an example of the constitution of a rotating stage 100 for controlling $\theta$-axis in the first embodiment. Two thick plates are connected by a connecting part 103 which functions as the supporting point of rotation and a connecting part 104 which functions as a spring so that one of two thick plates functions as a fixed part 101 and the other thick plate functions as a rotating part 102. This structure is produced, for example by wire-cutting one thick plate. A holding piece 105 is fixed on the side of the fixed part 101 close to the connecting part 104 by a supporting bolt 106. A piezoelectric element 108 is provided between the side of the rotating part 102 close to the connecting part 104 and the holding piece 105. The piezoelectric element 108 is set so that the rotating part 102 is in a state in which it is a little pressed on the right side of the drawing in a state in which control voltage is not applied. Therefore, for example, when the units 9 and 9' respectively consisting of the X-ray half mirrors are fixed on the upper surface of the rotating part 102 as shown by a broken line in Fig. 6 and the polarity and magnitude of voltage applied to the piezoelectric element 108 are controlled, the piezoelectric element 108 is stretched or contracted according to the polarity and magnitude of applied voltage, the rotating part 102 is displaced based upon the fixed part 101 in the direction shown by an arrow and the rotating part 102 can be rotated with the connecting part 103 in the center.

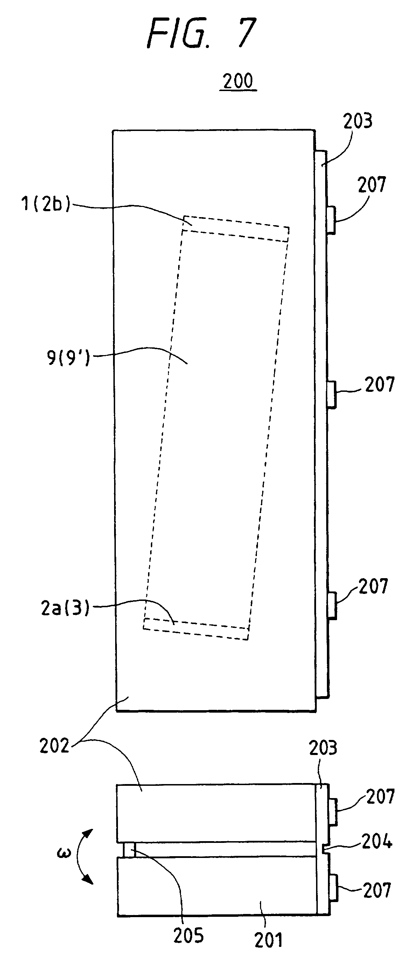

The fixed part 101 and the rotating part 102 are constituted so that the bottom of the rotating part 102 is a little lifted from the bottom of the fixed part 101 as the side view shows. Hereby, the rotating part 102 can be smoothly rotated. Further, each thick plate of the fixed part 101 and the rotating part 102 may be formed by an individual thick plate. In this case, the parts are required to be connected by a connecting piece provided with a supporting point and the function of a spring as described in relation to the constitution shown in Fig. 7 in place of the connecting parts 103 and 104.

Fig. 7 is a plan and a side view showing an example of the constitution of a rotating stage 200 for controlling $\omega$-axis in the first embodiment. The side of each thick plate is connected by a connecting piece 203 so that one of two thick plates functions as a fixed part 201 and the other thick plate functions as a rotating part 202. The connecting piece 203 connects both parts by a bolt 207 and a narrowed part 204 is formed in the center so that the connecting piece functions as the supporting point of rotation and is provided with the function of a spring. A piezoelectric element 205 is provided between opposite faces on the reverse side of the connecting piece 203 of each thick plate. The piezoelectric element 205 is set so that the rotating part 202 is a little lifted upward as shown in the side view in Fig. 7 from the lower surface of the drawing with no control voltage applied. Therefore, for example, when the units 9 and 9' respectively consisting of the X-ray half mirrors are fixed on the upper surface of the rotating part 202 as shown by a broken line and the polarity and magnitude of voltage applied to the piezoelectric element 205 are controlled, the piezoelectric element 205 is stretched or contracted according to the polarity and magnitude of applied voltage, the rotating part 202 is displaced based upon the fixed part 201 in the direction shown by an arrow and the rotating part 202 can be rotated with the narrowed part 204 of the connecting piece 203 as a supporting point.

As described above, in this embodiment, fine adjustment axes which are important to an X-ray half mirror are θ-axis and ω-axis and when they are individually adjusted, the stages shown in Figs. 6 and 7 may be used, however, if both θ-axis and ω-axis of one X-ray half mirror are to be adjusted, the stages shown in Figs. 6 and 7 are required to be heaped and hereby, both axes can be individually adjusted. For example, the fixed part 201 of the stage 200 shown in Fig. 7 is fixed on the movable part 102 of the stage 100 shown in Fig. 6 with the length and direction matched and the unit 9 or 9' consisting of the X-ray half mirrors is fixed on the upper surface of the movable part 202 of the stage 200 as shown by a broken line with the unit tilted by six degrees. The unit is required to be fixed with it tilted by six degrees because the unit 9 or 9' consisting of the X-ray half mirrors is carved from an ingot grown with the ingot tilted from the <111> axis to <110> axis by six degrees to use for reflection (440). As a result, for example, the movable part 102 of the lower stage 100 is controlled corresponding to θ-axis and the movable part 202 of the upper stage 200 is controlled corresponding to ω-axis.

Next, a method of obtaining diagnostic information based upon an interference pattern will be described. In the case of a conventional type method utilizing absorption contrast, a contrast is basically not varied depending upon an optical system and in addition, it is never inverted. It is because the projection of an X-ray absorption coefficient which is quantity proper to a substance determines the contrast of an image. In the case of a phase contrast method according to the present invention, an image with a contrast showing the distribution of quantity (a reflective index) proper to a substance is also obtained as an interference pattern if an optical system is ideally constituted, however, in the case of a phase contrast method, an image with a contrast showing the distribution of a reflective index is not always obtained and when such an image cannot be obtained, this image cannot be used for diagnosis. In the meantime, if an image showing the distribution of phase shift can be obtained when the projection of a reflective index is considered to be equivalent to X-ray phase shift, the image can be always used for diagnosis. Therefore, a method of obtaining an image showing the distribution of phase shift based upon an X-ray interference pattern is required. Some methods of determining phase shift based upon an interference pattern are established in the field of research on the interference of light and one of them which can be utilized for an X-ray interferometer is a fringe-scanning method (J. H. Bruning, et al., Appl. Opt., 33, 2693-2673 (1974)). This method is a method of obtaining an image showing the distribution of phase shift by calculation based upon plural interference patterns obtained by changing the relative phase difference of mutually interfering two X-ray beams by degrees. It is the principle of a fringe-scanning method that if the deflection angle of an expression,

$$\sum_{k=1}^{M} I_k \exp\left(-2\pi i \frac{k}{m}\right)$$

is calculated in case M pieces of interference patterns are obtained changing phase difference by $2\pi/M$, an image showing the distribution of phase shift can be obtained. In the above expression, $I_k$ denotes an interference pattern obtained when phase difference is set to $2\pi \frac{k}{M}$ and $i$ denotes an imaginary unit.

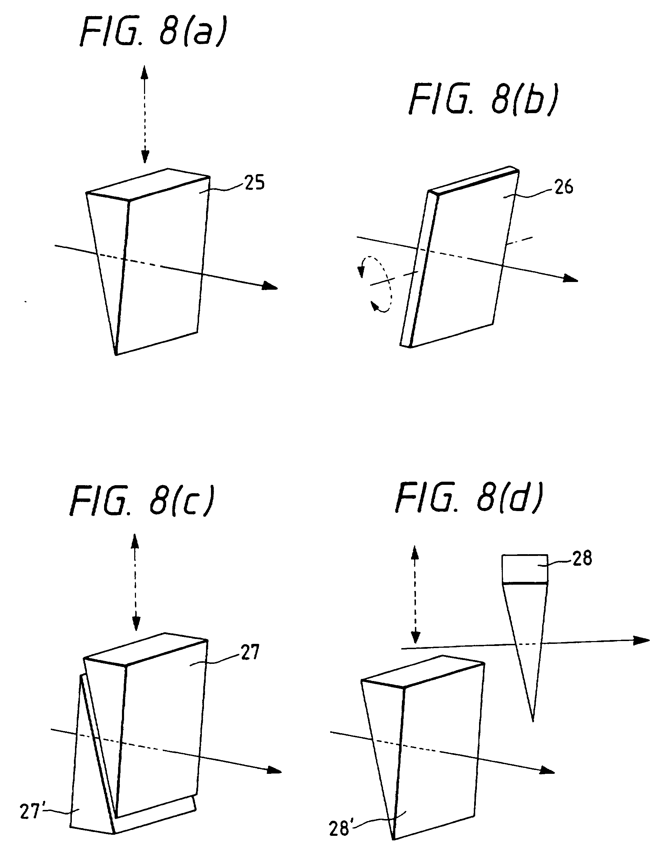

Figs. 8 show examples of phase plates arranged in the beam path of an X-ray interferometer for executing the fringe-scanning method and phase difference can be adjusted by moving them. Fig. 8 (a) utilizes a wedge of phase plate 25 for moving a beam path in the tilted direction of the wedge 25 by inserting it in one beam path of the X-ray interferometer. If the wedge 25 is moved as shown by an arrow in Fig. 7 because the quantity of phase shift by an X-ray phase plate is in proportion to the thickness of the X-ray phase plate, the thickness in the position where an X-ray is transmitted can be changed and phase difference proportional to the quantity of movement of the wedge 25 can be provided. However, the phase plate of this type generates phase grade in an X-ray beam and as a result, generates an interference pattern with an equal interval. There is no problem in the principle because a desired image (an image showing the distribution of the phase shift of a subject) is obtained if this phase grade is subtracted from an image showing the distribution of phase shift obtained using the above fringe-scanning method, however, if the precision of fringe scanning is bad, there is a defect that an interference pattern by the wedge 25 is left as a trace in an image showing the distribution of phase shift and a striped artifact is formed. Fig. 8 (b) shows a method of changing the thickness of a phase plate by turning the phase plate 26 as shown by an arrow and adjusting phase difference. As the phase plate 26 itself generates no interference pattern, there is no anxiety that an artifact is formed as in case the wedge-type phase plate 25 is used. Instead, as the turning angle of a phase plate is not in proportion to provided phase difference, the turning angle and phase difference are required to be calibrated beforehand. Fig. 8 (c) shows a state in which wedges in the same shape 27 and 27' are piled with anti-parallel arrangement and a method in which phase difference is adjusted by moving at least one wedge in the tilted direction. The method is a method provided with both advantages provided to (a) that the quantity of the movement of the wedge is in proportion to provided phase difference and provided to (b) that there is no anxiety that a striped artifact is formed. In any of the above (a), (b) and (c), the above phase plate may be inserted in any of beam paths 6a, 6b, 7a and 7b shown in Fig. 1. A phase plate shown in Fig. 8 (d) is also used for the same object as that in Fig. 8 (c), however, Fig. 8 (d) shows a method of inserting each of wedge-type phase plates 28 and 28' in the same shape in the same direction in two beam paths, for example beam paths 6a and 7a shown in Fig. 1. To adjust phase difference, at least one wedge has only to be moved in the tilted direction shown by an arrow.

The object of the present invention is to embody an X-ray interferometer wherein a thick X-ray beam can be utilized and distance between mirrors is long enough and to enable the present invention to be applied to a medical diagnosis apparatus according to a phase-contrast X-ray imaging method. As in some cases, an X-ray interference pattern cannot be utilized for diagnosis as it is, such an X-ray interference pattern is devised so that an image showing the distribution of phase shift can be obtained based upon the X-ray interference pattern and an image of the same contrast can be always provided without depending upon the degree of the adjustment of an apparatus. A new diagnostic method such as phase-contrast mammography and phase-contrast angiography can be realized using a system according to the present invention and further, phase-contrast X-ray computed tomography can be realized by obtaining an image showing the distribution of phase shift from plural directions of projection by rotating a subject and by processing the obtained images. A soft tissue such as a cancer in a living body which it is difficult to diagnose according to a conventional type method can be diagnosed at approximately a thousand times as much sensitivity by these means. The quantity of X-rays radiated on a body can also greatly reduced, compared with that according to the conventional type method. Further, as an X-ray beam through an X-ray interferometer is substantially a plane wave, an image is hardly dimmed and an image of 50 μm or less in space resolving power can be obtained.

Fig. 9 shows the whole constitution of a mammographic system constituted using the unit consisting of the X-ray half mirrors described in relation to Fig. 4. Each unit is fixed on stages 36 and 37. In this embodiment, the rotating stage for adjusting θ-axis shown in Fig. 6 is adopted as the stage 36 and the stage constituted by heaping the rotating stage for adjusting θ-axis shown in Fig. 6 and the rotating stage 200 for adjusting ω-axis shown in Fig. 7 for adjusting θ-axis and ω-axis as described above is adopted as the stage 37. As the adjustment of θ-axis and ω-axis is relative, the relative turning angle of the units 9 and 9' can be similarly adjusted even if the relationship between θ-axis and ω-axis is inverted. The stages 36 and 37 are arranged on a table 39 and further, the whole system is housed in a chamber 51, however, only the outside line of the chamber is shown by a reference number 51. The sides of the units 9 and 9' are mirrors and rough adjustment of the relative turning angles θ and ω of the unit 9' (the X-ray half mirrors 2b and 3) for the unit 9 (the X-ray half mirror 1 and 2a) is executed by an autocollimator 307 based upon light reflected on these mirrors prior to diagnosis. Light 308 emitted from the autocollimator 307 is reflected on the side of the unit 9 and returned and light 309 is reflected on the side of the unit 9' via rectangular prisms 310 and 311 and returned. A signal for roughly adjusting the angles θ and ω is obtained by examining positions in which the lights 308 and 309 are returned. Each mirror plane and each crystal lattice plane of the two units are produced so that the respective angles of each unit are as equal as possible, however, displacement between both units is examined using X-rays beforehand and rough adjustment is required to be executed in consideration of the displacement. As the two units are far, an optical path is changed using a prism and a mirror, however, an effect by an optical element at this time is measured beforehand and correction is required to be made. The operation of rough adjustment will be described later.

An X-ray incident on the X-ray half mirror 1 of the unit 9 is separated into beams 6a and 7a by an X-ray half mirror 1 and the beam 6a is separated into beams 6b and 6c by an X-ray half mirror 2a. The beam 7a is separated into beams 7b and 7c by an X-ray half mirror 2b. The beam 6b and 7b interfere by an X-ray half mirror 3 and are output as beams 6d and 7d. The checked part 50 of a subject 49 is put in the path of the beam 6b. At this time, a shield plate 53 is provided at a part of the chamber 51 on which the beam 6c is to be incident so that the beam is prevented from being incident on the subject 49. If the subject 49 is arranged in the path of the beam 7b, the same diagnosis is also enabled and in that case, a concave portion is provided on the side of the beam 7b. Prior to imaging an image, a checked part (a mamma) 50 is pressed in a predetermined position by a holder 54 so that the thickness of the checked part is fixed and for example, is temporarily fixed on a bed. However, it is desirable that the degree of freedom in which the holder can be a little moved in parallel and can be a little rotated is provided to the holder 54 and flexibility in imaging an image is secured. Reference numbers 55 and 56 respectively denote a phase plate and its driving base and the above phase plate and its driving base are installed in the path of the beam 7a so that when diagnosis is difficult in the shape of an interference pattern, diagnosis based upon an image showing the distribution of phase shift by a fringe-scanning method is enabled. The driving base 56 is provided to prevent vibration when driven from being transmitted to the base 39 and fixed on the ceiling of the chamber 51. The phase plate is driven by a signal from a controller 328 instructed by a computer 60. Reference numbers 57 and 58 denote X-ray intensity monitors and they are arranged so that they respectively receive the beam 7c and 7d. Further, a reference number 81 also denotes an X-ray intensity monitor, however, this is arranged in the end of a predetermined position of the beam 7a and receives X-rays at the end of the beam 7a. For example, PIN diode detector is used for these X-ray intensity monitors 57, 58 and 81 and as a result, a method of measuring current flowing when an X-ray is incident is facilitated. Rough adjustment prior to diagnosis is executed using the output of these X-ray intensity monitors 57, 58 and 81. The interference patterns obtained by the beams 6d and 7d of the checked part 50 are substantially the same and in this embodiment, the interference pattern is detected by the X-ray two-dimensional sensor 59 arranged so that the beam 7d can be received, however, the X-ray intensity monitor 58 may be also used as an X-ray two-dimensional sensor to obtain the X-ray interference pattern. In an example shown in Fig. 9, the X-ray intensity monitor 58 is used as the X-ray two-dimensional sensor and a signal from it is utilized for a feedback signal for stabilizing an interferometer differently from the X-ray interference pattern obtained by the X-ray two-dimensional sensor 59. The X-ray two-dimensional sensors 58 and 59 are driven by camera controllers 63' and 63 and the camera controllers 63' and 63 are controlled by the computer 60. An image is instructed to be imaged by a control program run in the computer 60 and an obtained image is stored in the memory of the computer 60 via the camera controllers 63' and 63. Diagnosis is executed based upon image data stored in this memory and a control signal for stabilization by feedback is output to the controller 325. A voltage controlled signal is applied to the piezoelectric element of the stage 37 in which the two stages are heaped by a signal from the controller 325 which receives an instruction from the computer 60, the feedback of $\theta$ and $\omega$ is executed, however, this concrete example will be described in detail later.

It is desirable that an X-ray incident to the X-ray half mirror 1 of the unit 9 is supplied from an X-ray source 33 arranged in a chamber isolated via a partition 52. It is because unnecessary radiation on the subject 49 can be prevented and vibration caused from the X-ray source 33 can be prevented from being transmitted to the X-ray interferometer.

A specific energy of X-ray beam 4 is extracted from an X-ray emitted from the X-ray source 33 using a monochromator 34 and transmitted to an image detector. The monochromator 34 simultaneously widens the width of the beam 4 by asymmetric reflection (the case of $0 < \alpha < \theta$ in Fig. 3 (a)). It is desirable that the diffraction index is the same as that of the X-ray half mirror and (220), (440), (400), (422) and others are desirable. The X-ray source 33 longer sideways as shown in Fig. 9 is advantageous in widening the width of a beam by the monochromator 34 and hereby, an intense X-ray beam is supplied into the interferometer. A shutter 35 is provided immediately after the monochromator 34 to prevent an X-ray from being radiated unnecessarily in other than imaging. This shutter 35 may be also installed immediately after the X-ray source 33.

Next, feedback control for rough adjustment prior to diagnosis and stabilizing the interferometer will be described.

First, the output from the X-ray intensity monitor 81 will be described. If 'θ' of the X-ray half mirror 1 is not suitable because the X-ray half mirror normally functions only when it meets a diffraction condition, an X-ray is not transmitted through the X-ray half mirror suitably. As the X-ray intensity monitor 81 is arranged at the end of the beam 7a, an X-ray is hardly sensed if 'θ' is not suitable. Therefore, when an X-ray is hardly sensed by the X-ray intensity monitor 81, a signal for correcting 'θ' so that the output from the X-ray intensity monitor 81 is substantially maximum is sent to the stage 36. Next, a signal for adjusting angles θ and ω is sent to the stage 37 so that the reflected light on the respective sides of the unit 9 (the X-ray half mirrors 1 and 2a) and the unit 9' (the X-ray half mirrors 2b and 3) of light 308 and 309 output from the autocollimator 307 is parallelized. After the above rough adjustment, the two piezoelectric elements of the stage 37 are controlled until an interference pattern is sensed by an X-ray image sensor 59 and an interference pattern is found by scanning two rotation axes (θ and ω). When an interference pattern is obtained, diagnosis can be started by controlling the two rotation axes (θ and ω) so that this interference pattern is optimum.

If θ-axis and ω-axis which require high precision adjustment are drifted even after an interference fringe is obtained and diagnosis can be started, an interference pattern is varied. Therefore, while a subject is diagnosed, the X-ray interferometer can be stabilized by monitoring an interference pattern obtained by the X-ray image sensor 58. When θ-axis and ω-axis are drifted, an interference pattern is varied, however, its state is different depending upon an axis. In the case of θ-axis, apparent phase difference between the two beams 6b and 7b is varied, in the meantime, in case ω-axis is drifted, a fringe like moire fringes is generated and is stretched or contracted depending upon the quantity of the drift of ω-axis. Therefore, stable diagnosis is enabled by feedback-controlling so that the change of an interference pattern obtained by X-ray image sensor 58 is deleted by processing by the computer 60 via the camera controller 63'.

When an image is required to be obtained by the fringe-scanning method, the control program run in the computer 60 instructs the driving base 56 of the phase plate and the camera controller 63 to obtain plural images (an interference pattern), changing phase difference. The computer 60 operates as shown in the expression (1) based upon the obtained images and the generated image showing the distribution of phase shift is displayed on the display of the computer 60.

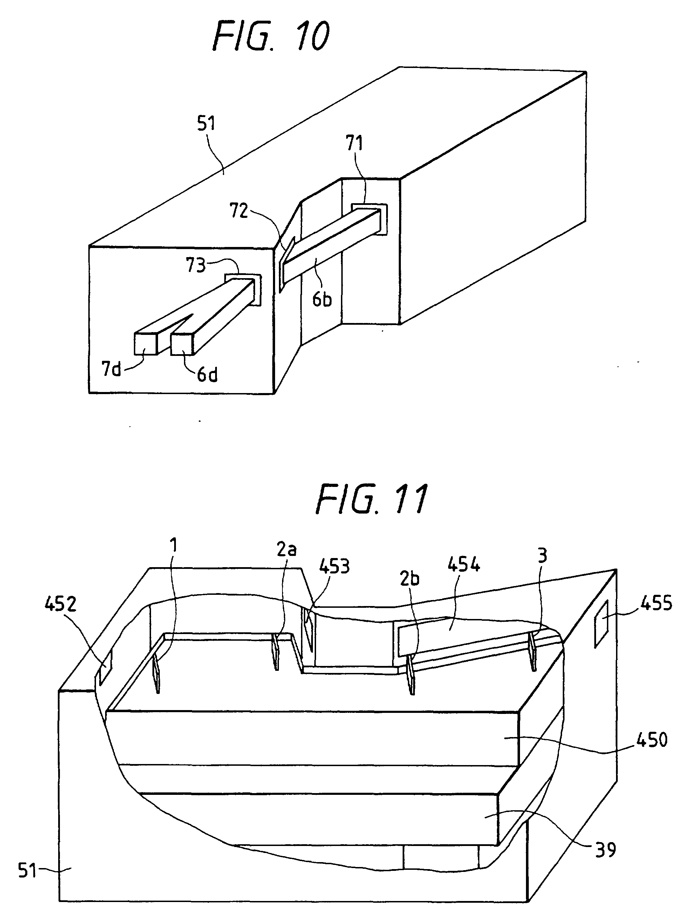

Fig. 10 is a perspective drawing showing the above constitution in the first embodiment in a state in which it is covered by the chamber 51. The beam 6b is once led outside the chamber 51 through a window 71, after the beam is transmitted through the checked part 50 not shown of the subject 49, it is again incident into the chamber 51 through a window 72, the interference beams 6d and 7d are led outside the chamber 51 through a window 73 and measured. The windows 71 to 73 for partitioning the chamber 51 and preventing heat generated by the subject 49 such as his/her body temperature and breath from having an effect upon an optical system are made of a plastic plate which does not absorb an X-ray so much and others. In Fig. 10, an incident X-ray is not shown.

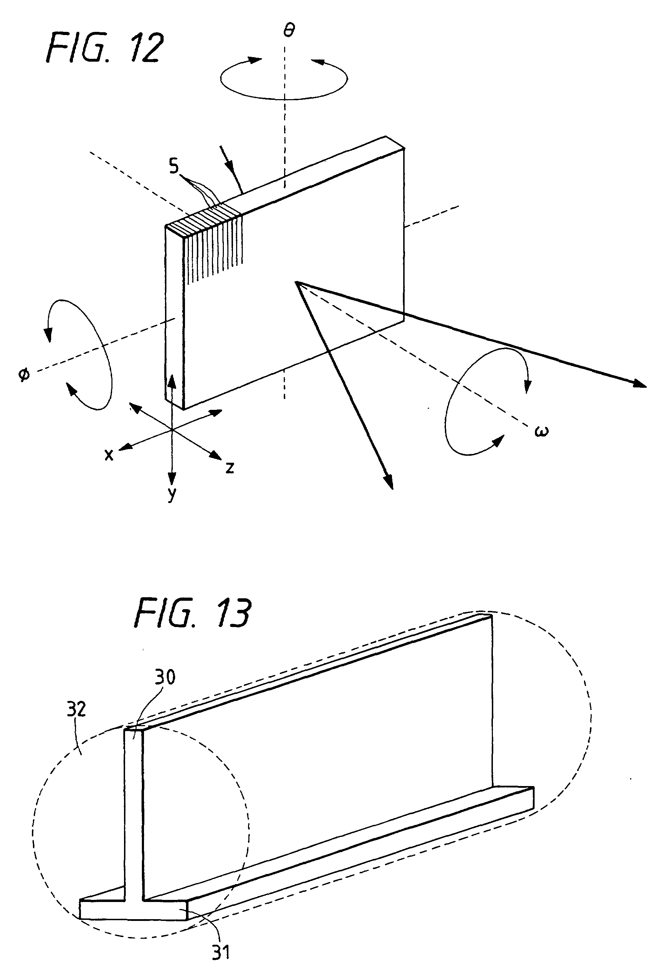

Fig. 11 shows the outline of an embodiment of an X-ray interferometer provided with the same constitution as in the above first embodiment for which a measure against vibration as faster fluctuation is taken.

As an X-ray interference pattern is spatially fluctuated at a high frequency when the units 9 and 9' respectively consisting of the X-ray half mirrors are relatively vibrated, the visibility of an interference pattern may be apparently deteriorated and an interference pattern may be invisible. Therefore, a device is required so that vibration is not transmitted to the units 9 and 9'. In this embodiment, as shown in Fig. 10, the X-ray half mirrors 1, 2a, 2b and 3 are respectively fixed in a pool 450, liquid provided with high viscosity such as oil is filled in the pool and the pool is constituted so that only the X-ray half mirrors 1, 2a, 2b and 3 appear over a liquid level. The pool 450 is put on the base 39 and the whole is covered by the chamber 51. Hereby, vibration of a high frequency component is prevented and the visibility of an interference pattern is enhanced. Plates made of beryllium are stuck on windows 452, 453, 454 and 455 provided on the walls of the chamber 51 through which X-rays pass to prevent outside air from flowing inside the chamber. In addition, a polymer film, a thin aluminum plate and a glass plate can be used.

This embodiment is an example in which the part of an interferometer under the X-ray half mirrors is sunk in liquid with high viscosity in addition to a sound-proof chamber, a sound insulating wall and a base normally used.

In the first embodiment, if the pool as shown in Fig. 11 is not used, the room of chamber 51 can be made substantially vacuum so that it prevent to transfer for temperature regulation etc. of surrounding to X-ray half mirrors.

Second Embodiment

In this embodiment, an X-ray interferometer is constituted by independent three X-ray half mirrors 1 to 3 as shown in Fig. 2 (a). Fig. 12 shows the degree of freedom required in case X-ray half mirrors are adjusted in this embodiment. The case of an X-ray reflecting mirror is also similar. As described in relation to the first embodiment shown Fig. 4, a thick full line shown by an arrow in Fig. 12 shows an X-ray which is incident on the center of a half mirror and outgoing from the center. A reference number 5 representatively denotes a part of a crystal diffraction lattice plane, the direction of the normal of the diffraction lattice plane 5 is x-axis, the direction of the normal of a scattering plane (a surface including an arrow showing the propagation direction of an X-ray beam in Fig. 12) is y-axis and an axis perpendicular to x-axis and y-axis is z-axis. Rotation axes around x-, y- and z-axes are respectively $\phi$-axis, $\theta$-axis and $\omega$-axis. For the parallel movement of an X-ray half mirror, the precision of x-axis is severely required and precision of 1/a few Å or less is required. For z-axis, precision of 1 $\mu$m is enough and for y-axis, particularly severe fine adjustment is not required. For rotation, $\theta$-axis is an axis related to Bragg diffraction condition and precision of one tenth second or less is required. For $\omega$-axis orthogonal to $\theta$-axis and perpendicular to a crystal surface, precision of one hundredth second or less is required. For $\phi$-axis, particular severe fine adjustment is not required. Therefore, important axes to be finely adjusted are x-, z-, $\omega$- and $\theta$-axes.

As shown in Fig. 13, each X-ray half mirror 1 to 3 is carved from a cylindrical silicon ingot 32 while being divided longitudinally. A crystalline plate 30 which functions as an X-ray half mirror is carved from an ingot 32 shown by a broken line so that it is integrated with a base 31. It is desirable that the diffraction surface of X-ray half mirrors 1 to 3 is (220), (440), (400), (422) and others.

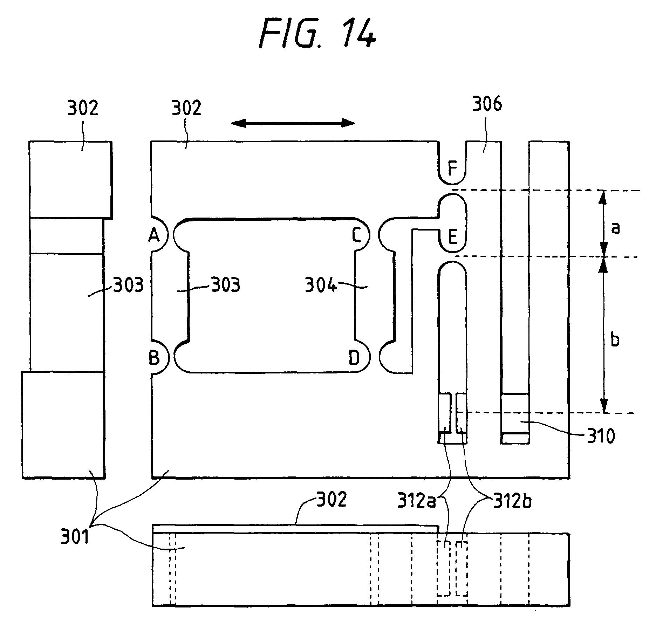

Fig. 14 is a plan in the center and side views on the right and left sides showing an example of the constitution of a movable stage in the direction of x-axis 300 for embodying positional control of 1/a few Å or less. The stage 300 is a movable stage parallel to one axis and is carved from a thick steel plate by wire cutting. A stage attachment part 301 and a movable part 302 are arranged in parallel and coupled by supporting pieces 303 and 304. The supporting pieces 303 and 304 are coupled via the respective narrowed parts A to D. A reverse L-shaped piezoelectric element attachment part 305 is provided at one end of the stage attachment part 301. One end of a lever 306 is coupled to one end of the movable part 302 via a narrow part F. A piezoelectric element 310 is provided between the other end of the lever 306 and the piezoelectric attachment part 305. A narrow part E is formed close to the narrow part F, one end is supported by the lever 306 and the end is supported by the stage attachment part 301. Electrodes 312a and 312b are provided between the surface reverse to the surface on which a piezoelectric element 310 is provided of the lever 306 and the stage attachment part 301. The narrow parts A to F can be elastically transformed. When the polarity of magnitude of voltage applied to the piezoelectric element 310 are controlled, the piezoelectric element 310 is stretched or contracted according to the control and the movable part 302 is displaced in the direction shown by an arrow. As points E and F are equivalent to a supporting point and a point of application at that time, the length of the stretched or contracted piezoelectric element 310 is reduced by this principle and the position of the movable part 302 can be finely controlled. Reduction ratio is determined by selecting the ratio of distance a/b. The quantity of stretched or contracted piezoelectric element 310 can be monitored by measuring electrostatic capacity between the electrodes 312a and 312b. As known from both side views shown in Fig. 14, the respective bottoms of the movable part 302, the supporting pieces 303 and 304 and the lever 306 are extremely a little higher than the bottom of the stage attachment part 301 and further, the upper surface of the movable part 302 is extremely a little higher than the respective upper surfaces of other parts. Hereby, the movable part 302 can be smoothly moved and when stages are heaped, the movable part can be also controlled without difficulty.

In this embodiment, the control of ω-axis and θ-axis is also required and a rotating stage which can be utilized for it is required, however, as the rotating stages shown in Figs. 6 and 7 can be used for the respective rotation axes, an example of the constitution of a-rotating stage in this embodiment is not shown in the drawing.

As shown in Fig. 12, as important axes to be finely adjusted for X-ray half mirrors are x-, z-, ω- and θ-axes, the stages shown in Figs. 14, 6 and 7 are heaped and the heaped stages can support an independent X-ray half mirror with them adjustable. For example, assume that the stage shown in Fig. 14 is shifted by 90° and heaped vertically, the stage attachment part 301 of the lower stage is fixed on the base common to independent each X-ray half mirror and the stage attachment part 301 of the upper stage is fixed on the movable part 302 of the lower stage. If for example, the movable part of the lower stage is controlled in the direction of x-axis, the movable part of the upper stage is controlled in the direction of z-axis. Similarly, if the rotating stages shown in Figs. 6 and 7 are heaped as in the first embodiment, ω-axis and θ-axis can be also controlled in this embodiment. If a thick plate 101 on the fixed side of the lower stage of the rotating stages shown in Figs. 6 and 7 is fixed on the movable part of the upper stage of the stage shown in Fig. 14, the thick plate 202 on the rotating side of the stage shown in Fig. 7 is controlled corresponding to four axes of x-, z-, ω- and θ-axes.

In this embodiment differently from the first embodiment, independent X-ray half mirrors 1 to 3 are required to be individually controlled. A stage 36 for adjusting θ-axis is provided to the X-ray half mirror 1, a stage 37 heaped in two stages for adjusting θ-axis and ω-axis is provided to the X-ray half mirror 2, a piezoelectric stage 38 heaped in four stages consisting of the above rotating stages for adjusting θ-axis and ω-axis and stages movable in parallel to x-axis and y-axis is provided to the X-ray half mirror 3 and the above stages are arranged on the base 39 via these. A laser interferometer 42 is also arranged on the base 39 to detect displacement in the direction equivalent to x-axis in Fig. 12. A laser beam 45 emitted from the laser interferometer 42 is reflected on a reflecting mirror 48 such as a corner cube fixed on the piezoelectric element stage 38 and returned to the laser interferometer 42. A concave portion is provided on the base 39 so that the checked part 50 of a subject 49 can be put in the path 6b of an X-ray beam. If a subject 49 is arranged in the path of the beam 7b, similar observation is also enabled and in that case, a concave portion is provided on the side of the beam 7b. For stabilizing the operation of an X-ray interferometer and a laser interferometer and the safety of a subject 49, a chamber 51 according to the shape of the base 39 is provided to prevent the X-ray interferometer and the laser interferometer from being in contact with the outside, an X-ray shield 53 is provided to prevent X-rays other than required X-rays from being radiated on a subject 49 and prior to imaging, a checked part (mamma) 50 is pressed by a holder 54 in a predetermined position so that the thickness is fixed and for example, is temporarily fixed on a bed. However, it is the same as in the first embodiment that the degree of freedom which enables a little parallel movement and a little rotation is provided to the holder 54 and it is desirable to secure flexibility in imaging. Reference numbers 55 and 56 respectively denote a phase plate and its driving base and when diagnosis as an interference pattern is difficult, they are arranged in the path of the beam 7a as in the first embodiment so that diagnosis based upon an image showing the distribution of phase shift is enabled by the fringe-scanning method. The phase plate is driven by a signal from a controller 328 which receives an instruction from a computer 60. Reference numbers 57, 58 and 81 are intensity monitors, arranged as in the first embodiment and used for rough adjustment of the X-ray half mirrors 1 to 3. Substantially the same interference patterns of the checked part 50 are observed in the paths of beams 6d and 7d, however, in this embodiment as in the first embodiment, an X-ray two-dimensional sensor 59 arranged so that it can receive the beam 7d also detects an interference pattern.

The rough adjustment of the X-ray half mirrors 1 to 3 by the intensity monitors 57, 58 and 81 will be described below. In this embodiment as in the first embodiment, 'θ' of the X-ray half mirror 1 is also roughly adjusted so that the output of the intensity monitor 81 is substantially maximum. Next, 'θ' of the X-ray half mirror 2 is roughly adjusted so that the output of the intensity monitor 57 is substantially maximum. Afterward, 'θ' of the X-ray half mirror 3 is roughly adjusted so that the output of the intensity monitor 58 is substantially maximum.

After the above rough adjustment, two rotation axes θ-axis and ω-axis are scanned controlling the two piezoelectric elements of the stage 37 until an interference pattern is sensed by an X-ray image sensor 59 and two rotation axes θ-axis and ω-axis and z-axis are scanned controlling the three piezoelectric elements of the stage 38 to find an interference pattern. When an interference pattern is once obtained, the two rotation axes θ-axis and ω-axis are controlled so that this interference pattern is optimum and diagnosis can be started.

The X-ray two-dimensional sensors 58 and 59 are driven by camera controllers 63' and 63 and the camera controllers 63' and 63 are controlled by the computer 60. Imaging is instructed by a control program run in the computer 60 and obtained images are stored in the memory of the computer 60 via the camera controllers 63' and 63. Diagnosis is performed based upon image data stored in the memory and a control signal for stabilization by feedback is sent to a controller 325. A voltage controlled signal is applied to the respective piezoelectric elements of the stage 36, the stage 37 heaped in two stages and the stage 38 heaped in four stages according to a signal from the controller 325 which receives an instruction from the computer 60 and the feedback of θ-, ω- and x-axes is executed, however, this concrete example will be described later.

When θ-, ω- and x-axes which require high precision adjustment are drifted after an interference pattern is once obtained and diagnosis can be started, an interference pattern is varied. As in the first embodiment, while a subject is diagnosed, the X-ray interferometer can be stabilized by monitoring an interference pattern obtained by the X-ray image sensor 58. The drift of θ-axis and ω-axis is described in relation to the first embodiment and when x-axis is also drifted as θ-axis, apparent phase difference between the two beams 6b and 7b is varied. Therefore, stable diagnosis is enabled by feedback-controlling so that the change of an interference pattern obtained by the X-ray image sensor 58 is deleted by processing by the computer 60 via the camera controller 63'.

When an image is required to be obtained by the fringe-scanning method, it is desirable that an image showing the distribution of phase shift generated as in the first embodiment is displayed on the display of the computer 60.

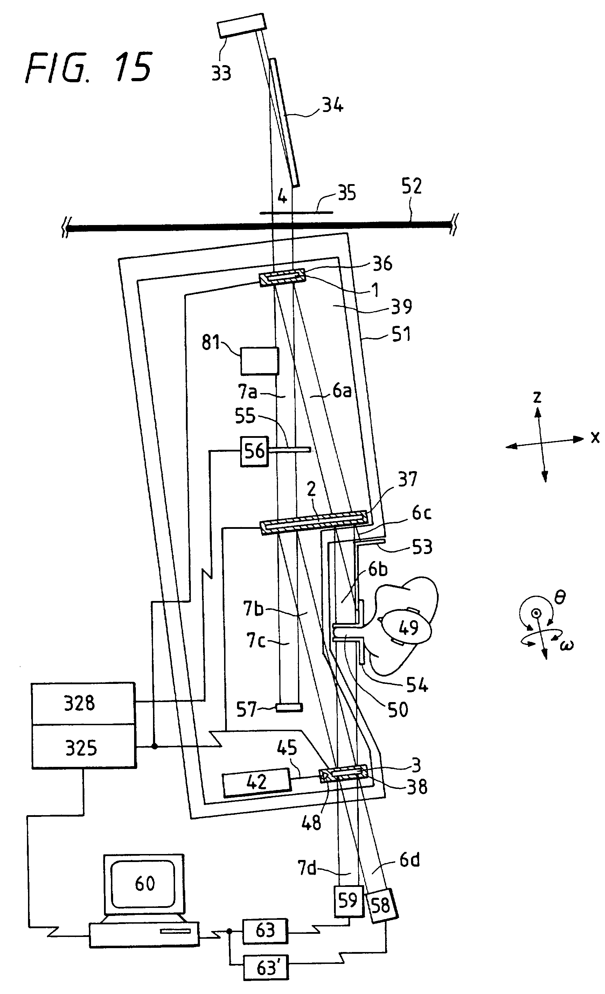

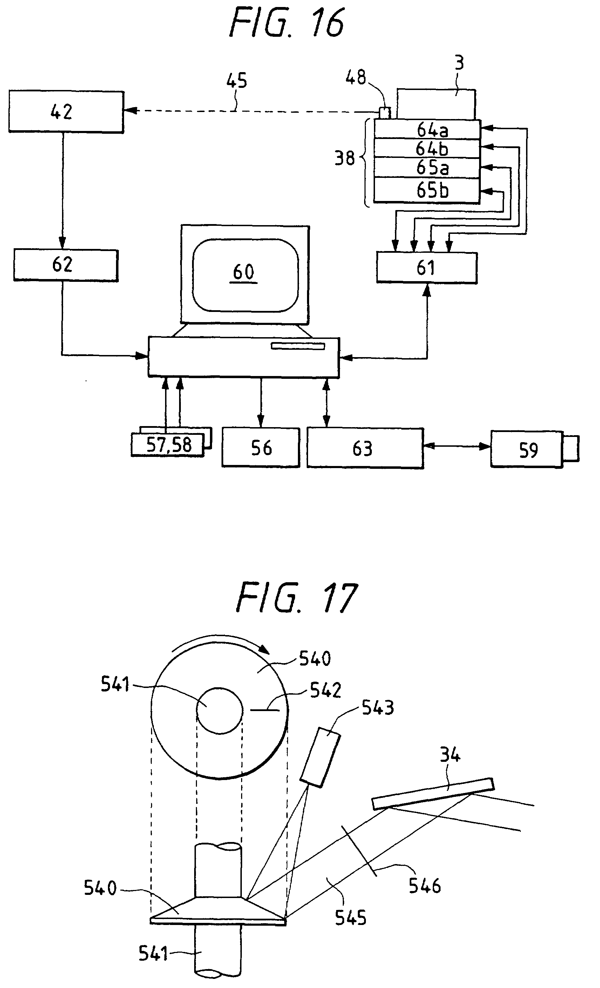

Fig. 16 is a block diagram when the stage 38 heaped in four stages of an apparatus shown in Fig. 15 is driven and controlled by a computer. The control of the stages 36 and 37 is described in relation to the first embodiment and the description is omitted. The piezoelectric element stage 38 for supporting the X-ray half mirror 3 is constituted by combining the parallel movable stages 65a and 65b provided with the structure shown in Fig. 14 and the rotating stages 64a and 64b provided with the structure shown in Figs. 6 and 7 as described above to adjust x-, z-, ω- and θ-axes described in relation to Fig. 12. The piezoelectric element stage 38 is driven by a piezoelectric element controller 61 provided with plural channels. When the piezoelectric element stage 38 is driven by output from the piezoelectric element controller 61, the piezoelectric element controller 61 reads the change of the output of electrostatic capacity sensors 312a and 312b corresponding to the movement of the movable part of the parallel movable stages 65a and 65b and outputs voltage generated by correcting the hysteresis of the piezoelectric element to the piezoelectric element. The output of the sensor is also sent to the computer 60. The laser interferometer 42 emits a laser beam 45, the reflecting mirror 48 installed together with the X-ray half mirror 3 on the piezoelectric element stage 38 reflects the laser beam 45 and returns it to the laser interferometer 42. When the position (in the direction of x-axis) of the piezoelectric element stage 38 is changed, the laser interferometer 42 detects variation, outputs a signal including the information of the variation and sends it to the computer 60 via an A/D converter 62. A control program run in the computer 60 processes the sent signal and instructs the piezoelectric element controller 61 to change voltage applied to the piezoelectric element as a feedback signal. Signals from the intensity monitors 57 and 58 are used for adjusting a feedback signal as the auxiliary data of the control program run in the computer 60. The X-ray two-dimensional sensor 59 is driven by the camera controller 63 and the camera controller 63 is controlled by the computer 60. Imaging is instructed by the control program run in the computer 60 and obtained images are stored in the memory of the computer 60 via the camera controller 63.

When an image is required to be obtained by the fringe-scanning method, plural images (interference patterns) are obtained changing phase difference as in the first embodiment and the computer 60 operates based upon the obtained images as shown in the expression (1) and the generated image showing the distribution of phase shift is displayed on the display of the computer 60.

As known from the contrast of Figs. 15 and 16, in Fig. 16 a part of lines showing the transmission of a signal to the computer for control and the transmission of a control signal from the computer for control to each stage are omitted to simplify the drawing.

In this embodiment, a stage for adjustment is provided to each X-ray half mirror 1 to 3, however, as the adjustment of these mirrors is relative, it need hardly be said that one of the three mirrors can be fixed.

As an transformed example of the first and second embodiments, a subject 49 may be put in the path of a beam 7a. In this case, there is a merit that the shield plate 53 required in the second embodiment can be omitted. As the quantity of X-rays radiated on a subject 49 is only doubled in the present invention even if the shield plate 53 is omitted, the quantity in which X-rays are radiated is by far smaller than that in the conventional type method. However, as the smaller the quantity of X-rays radiated on a subject is, the better it is and substantially the same performance can be expected for the X-ray interferometer even if the side on which an X-ray is incident and the side from which an X-ray is outgoing are inverted, a subject 49 may be put in the path of the beam 7a. Similarly, in the first embodiment, a subject may be also put in the path of the beam 6a. However, in these cases, as an X-ray which passes through the checked part 50 passes through the two X-ray half mirrors 2 and 3 until it reaches the two-dimensional detector 59, the spatial resolving power of an obtained image is a little deteriorated. In this embodiment, space which a subject 49 can use is extended and if is easy for a subject 49 to hold a posture in imaging. The size of the whole apparatus can be also reduced. A phase plate for applying a fringe-scanning method may be inserted in any path.

Fig. 17 shows an example of an X-ray source convienient for an X-ray interferometer.

As an X-ray interferometer functions for a quasi-monochrome X-ray, it is advantageous to use an intense X-ray source to some extent so as to image an image promptly. In the case of an optical system used in the above embodiments, the shape of the X-ray source is required to be thin in the perpendicular direction, however, may be long in the horizontal direction. Therefore, if a radiation source particularly excited by an electron beam or by a laser beam is used, it is effective to constitute the radiation source as shown in Fig. 17.

As shown in Fig. 17, a reference number 540 denotes a target, 541 denotes a rotation axis, 542 denotes an X-ray generated part, 543 denotes an electron beam source or a laser beam source, 544 denotes an electron beam or a laser beam, 545 denotes an X-ray and 546 denotes a filter. In Fig. 17, the plan of the target 540 is also shown. According to this constitution, an electron beam or a laser beam 544 linearly scans on the target 540 and an X-ray source longer sideways can be formed. Hereby, as heat provided to the target can be relatively diffused, more X-rays can be let to the X-ray interferometer.

Though the drawing is omitted, it is also effective that the direction in which a crystal 34 is reflected is differentiated in each embodiment and a monochrome apparatus in which two or more crystals are continuously reflected is used so as to supply a better X-ray beam to an X-ray interferometer.

In the above embodiments as shown in Figs. 9 and 15, we did not referred about spacer for adjusting the height of the half mirror. However the height of the half mirror should be adjusted by a spacer according to the number of a piezoelectric element driving stage.

According to the present invention, as an image can be sensitively imaged, contrast media are not required to be injected properly, however, when the contrast of a specific interested part is to be emphasized, contrast media may be used. In this case, contrast media consisting of heavy elements is not required to be used as in the conventional type method and contrast media consisting of a wide range of material can be used.

According to the present invention, a high sensitive phase-contrast X-ray imaging system provided with a wide visual field can be obtained.

**Claims**

1. A phase-contrast X-ray imaging system comprising

   an interferometer including
   a first half-mirror (1) for separating an incident X-ray into two mutually interfering beams, and
   a second half-mirror (3) for connecting a beam obtained by inserting an object (50) in the path of one (6b) of said two beams and the other beam (7b),
   a detector (59) for detecting a beam connected by said second half-mirror (3), and
   a processing section (60) for obtaining the image of said object (50) based upon the output of said detector (59),

   wherein at least one of both half-mirrors (1, 3) is installed on a table (39) via a mirror adjusting mechanism (40, 41) for adjusting the relative positional relationship between the half-mirrors (1, 3), so that an observation field exceeding 2 cm × 2 cm is obtained,
   **characterised in that** said X-ray interferometer is installed in a chamber (51), said chamber having a concave section for enabling one (6b) of the interfering X-ray beams to travel outside the chamber and pass through a portion of the object (50) at the concave section outside said chamber.

2. The system of claim 1, wherein said X-ray interferometer is constituted by two crystal blocks which each are monolithically cut out from ingots (32) of crystal and have two wafers which function as X-ray half mirrors, said crystal blocks being placed on stages provided on a common table in said chamber (51) and having mechanisms for adjusting the relative position between said two crystal blocks to generate interference.

3. The system of claim 1 or 2, wherein a tunable phase shifter is placed in the path of at least one of the interfering X-ray beams, an image showing the distribution of an X-ray phase shift caused by the object being generated from X-ray interference patterns of the interfering X-ray beams.

4. The system of claim 2, further comprising a pool in which said X-ray interferometer is placed, said pool being filled with a liquid of high viscosity so that said wafers of said crystal blocks are above the liquid surface of said pool.

5. The system of claim 4, wherein said stages for adjustment are also provided in said pool.

6. The system of any preceding claim, further comprising equipment for pumping air from the inside of said chamber (51).

7. The system of claim 2, further comprising optical equipment used for preliminary alignment of the X-ray interferometer constituted by said crystal blocks so that said two crystal blocks are placed to a proper position to generate interference by viewing polished facets of said two crystal blocks with the optical equipment.

8. The system of claim 2, including beam intensity monitors to adjust said crystal blocks, and image sensors to observe interference patterns which enable both diagnosing of the object and providing feedback signals which are sent to said stages for adjustment so that drifts in interference fringes are compensated.

9. The system of claim 1, wherein
said X-ray interferometer includes two crystal blocks which each are monolithically cut out from ingots (32) of crystal and have two wafers which function as X-ray half mirrors; comprising
stages for adjusting the two crystal blocks to a proper position where interference is generated;
optical equipment for preliminary alignment of the two crystal blocks by placing said two crystal blocks substantially to a proper position which satisfies a diffraction condition of said X-ray half mirrors by viewing polished facets of said two crystal blocks with the optical equipment;
X-ray intensity monitors for adjustment of the two crystal blocks to satisfy the diffraction condition of said X-ray half mirrors;
a tunable X-ray phase shifter placed in the path of at least one of the interfering X-ray beams for obtaining an image showing the distribution of an X-ray phase shift caused by the object placed on one of the paths of the interfering X-ray beams;
said stages for adjustment, said table, said X-ray intensity monitors, and said tunable X-ray phase shifter being disposed in said chamber (51),
X-ray image sensors for obtaining X-ray interference patterns of the interfering X-ray beams; and
a computer for controlling said tunable X-ray phase shifted, said stages for adjustment, and said X-ray image sensors, for analysing observed X-ray interference patterns to provide feedback signals which are sent to the stages for adjustment so that drifts in interference fringes are compensated.

**Patentansprüche**

1. Phasenkontrast-Röntgenabbildungssystem, umfassend
ein Interferometer mit
einem ersten Halbspiegel (1) zum Aufteilen eines einfallenden Röntgenstrahls in zwei miteinander interferierende Strahlen, und
einem zweiten Halbspiegel (3) zum Verbinden eines Strahls, der durch Einfügen eines Gegenstands (50) in den Pfad eines (6b) der beiden Strahlen entsteht, mit dem anderen Strahl (7b),
einen Detektor (59) zum Erfassen eines von dem zweiten Halbspiegel (3) verbundenen Strahls, und
einem Verarbeitungsteil (60) zum Erzeugen des Bildes des Gegenstandes (50) aufgrund des Ausgangssignals des Detektors (59),
wobei mindestens einer der beiden Halbspiegel (1, 3) auf einem Tisch (39) über einen Spiegeljustiermechanismus (40, 41) zum Justieren der relativen Positionsbeziehung zwischen den Halbspiegeln (1, 3) montiert ist, so daß ein Beobachtungsfeld von über 2 cm $\times$ 2 cm erhalten wird,
**dadurch gekennzeichnet, daß** das Röntgeninterferometer in einer Kammer (51) mit einem konkaven Abschnitt untergebracht ist, der es gestattet, daß einer (6b) der interferierende Röntgenstrahlen außerhalb der Kammer verläuft und einen Teil des Gegenstands (50) in dem konkaven Abschnitt außerhalb der Kammer durchsetzt.

2. System nach Anspruch 1, wobei das Röntgeninterferometer aus zwei Kristallblöcken aufgebaut ist, die jeweils von Kristallrohblöcken (32) monolithisch ausgeschnitten sind und zwei als Röntgenhalbspiegel wirkende Wafer aufweisen, wobei die Kristallblöcke auf Gestellen plaziert sind, die auf einem gemeinsamen Tisch in der Kammer (51) angeordnet sind und Mechanismen zum Justieren der relativen Position zwischen den beiden Kristallblöcken zum Erzeugen von Interferenz aufweisen.

3. System nach Anspruch 1 oder 2, wobei in dem Pfad mindestens eines der interferierenden Röntgenstrahlen ein abstimmbarer Phasenschieber angeordnet ist, und wobei aus Röntgeninterferenzmustern der interferierenden Röntgenstrahlen ein Bild erzeugt wird, das die Verteilung einer durch den Gegenstand bewirkten Röntgenphasenverschiebung zeigt.

4. System nach Anspruch 2 mit ferner einem Becken, in dem das Röntgeninterferometer angeordnet ist und das mit einer Flüssigkeit hoher Viskosität derart gefüllt ist, daß sich die Wafer der Kristallblöcke oberhalb des Flüssigkeitsspiegels in dem Becken befinden.

5. System nach Anspruch 4, wobei die Justiergestelle ebenfalls in dem Becken angeordnet sind.

6. System nach einem der vorhergehenden Ansprüche mit ferner einer Einrichtung zum Abpumpen von Luft aus dem Innern der Kammer (51).

7. System nach Anspruch 2 mit ferner einer zum Vorjustieren des Röntgeninterferometers dienenden optischen Einrichtung, die aus den Kristallblöcken derart gebildet ist, daß die Kristallblöcke in eine geeignete Stellung gebracht werden, um bei Beobachtung polierter Facetten der beiden Kristallblöcke mit der optischen Einrichtung Interferenz zu erzeugen.

8. System nach Anspruch 2 mit Strahlintensitätsmonitoren zum Justieren der Kristallblöcke sowie Bildsensoren zum Beobachten von Interferenzmustern, die sowohl eine Diagnose des Gegenstands als auch die Erzeugung von Rückkopplungssignalen gestatten, die den Justiergestellen zugeführt werden, so daß Verschiebungen in Interferenzstreifen kompensiert werden.

9. System nach Anspruch 1, wobei
das Röntgeninterferometer zwei Kristallblökke aufweist, die jeweils aus Kristallrohblöcken monolithisch ausgeschnitten sind und Wafer aufweisen, die als Röntgenhalbspiegel wirken, umfassend
Gestelle zum Justieren der beiden Kristallblöcke auf eine geeignete Position, in der Interferenz entsteht,
eine optische Einrichtung zum Vorjustieren der beiden Kristallblöcke derart, daß diese im wesentlichen in eine geeignete Position gebracht werden, die eine Brechungsbedingung der Röntgenhalbspiegel bei Beobachten polierter Facetten der beiden Kristallblöcke mit der optischen Einrichtung erfüllt,
Röntgenintensitätsmonitoren zum Justieren der beiden Kristallblöcke derart, daß die Beugungsbedingung der Röntgenhalbspiegel erfüllt wird,
einen im Pfad mindestens eines der interferierenden Röntgenstrahlen angeordneten abstimmbaren Röntgenphasenschieber zum Erzielen eines Bildes, das die Verteilung einer durch Einfügen des Gegenstands in einen der Pfade der interferierenden Röntgenstrahlen bewirkten Phasenverschiebung zeigt,
wobei die Justiergestelle, der Tisch, die Röntgenstrahl-Intensitätsmonitoren und der abstimmbare Röntgenphasenschieber in der Kammer (51) angeordnet sind,
ferner Röntgenbildsensoren zum Erzeugen von Röntgeninterferenzmustern der interferierenden Röntgenstrahlen und
einen Computer zum Steuern des abstimmbaren Röntgenphasenschiebers, der Justiergestelle und der Röntgenbildsensoren, um beobachtete Röntgeninterferenzmuster zu analysieren und dadurch Rückkopplungssignale zu erzeugen, die den Justiergestellen zugeführt werden, um Verschiebungen in Interferenzstreifen zu kompensieren.

## Revendications

1. Système d'imagerie à rayons X à contraste de phase, comportant :

un interféromètre incluant
un premier demi-miroir (1) pour séparer des rayons X incidents en deux faisceaux mutuellement interférents, et
un second demi-miroir (3) pour connecter un faisceau obtenu en insérant un objet (50) dans le trajet d'un premier (6b) desdits deux faisceaux et de l'autre faisceau (7b),
un détecteur (59) pour détecter un faisceau connecté par ledit second demi-miroir (3), et
une section de traitement (60) pour obtenir l'image dudit objet (50) sur la base de la sortie dudit détecteur (59),
au moins un des deux demi-miroirs (1, 3) étant installé sur une table (39) via un mécanisme d'ajustement de miroir (40, 41) destiné à ajuster la relation positionnelle relative entre les demi-miroirs (1, 3), de sorte qu'un champ d'observation dépassant 2 cm × 2 cm est obtenu,

**caractérisé en ce que** ledit interféromètre à rayons X est installé dans une chambre (51), ladite chambre ayant une section concave pour permettre au premier (6b) des faisceaux de rayons X interférents de se déplacer à l'extérieur de la chambre et de passer à travers une partie de l'objet (50) dans la section concave à l'extérieur de ladite chambre.

2. Système selon la revendication 1, dans lequel ledit interféromètre à rayons X est constitué de deux blocs cristallins qui sont chacun découpés de manière monolithe à partir de lingots (32) de cristal et ont deux plaquettes qui fonctionnent comme des demi-miroirs à rayons X, lesdits blocs cristallins étant placés sur des platines agencées sur une table commune de ladite chambre (51) et ayant des mécanismes pour ajuster la position relative entre lesdits deux blocs cristallins afin de générer une interférence.

3. Système selon la revendication 1 ou 2, dans lequel un élément à déphasage accordable est placé dans le trajet d'au moins un des faisceaux de rayons X interférents, une image représentant la distribution d'un déphasage de rayons X provoqué par l'objet qui est généré à partir des motifs d'interférence de rayons X des faisceaux de rayons X interférents.

4. Système selon la revendication 2, comportant de plus un bassin dans lequel ledit interféromètre à rayons X est placé, ledit bassin étant rempli d'un liquide ayant une viscosité élevée de sorte que lesdites plaquettes desdits blocs cristallins sont situées au-dessus de la surface de liquide dudit bassin.

5. Système selon la revendication 4, dans lequel lesdites platines d'ajustement sont également agencées dans ledit bassin.

6. Système selon l'une quelconque des revendications précédentes, comportant de plus un équipement pour pomper de l'air depuis l'intérieur de ladite chambre (51).

7. Système selon la revendication 2, comportant de plus un équipement optique utilisé pour un alignement préliminaire de l'interféromètre à rayons X constitué desdits blocs cristallins de sorte que lesdits deux blocs cristallins sont placés à une position correcte pour générer une interférence en observant des facettes polies desdits deux blocs cristallins à l'aide de l'équipement optique.

8. Système selon la revendication 2, comportant des dispositifs de surveillance d'intensité de faisceau pour ajuster lesdits blocs cristallins, et des capteurs d'image pour observer des motifs d'interférence qui permettent à la fois de diagnostiquer l'objet et de délivrer des signaux de rétroaction qui sont envoyés auxdites platines pour un ajustement de manière à compenser des dérives de franges d'interférence.

9. Système selon la revendication 1, dans lequel ledit interféromètre à rayons X comporte deux blocs cristallins qui sont chacun découpés de manière monolithique à partir de lingots (32) de cristal et ont deux plaquettes qui fonctionnent comme des demi-miroirs à rayons X, comportant

des platines pour ajuster les deux blocs cristallins à une position correcte où une interférence est générée,

un équipement optique pour un alignement préliminaire des deux blocs cristallins en plaçant lesdits deux blocs cristallins sensiblement à une position correcte qui satisfait à une condition de diffraction desdits demi-miroirs à rayons X en observant des facettes polies desdits deux blocs cristallins à l'aide de l'équipement optique,

des dispositifs de surveillance d'intensité de rayons X pour l'ajustement des deux blocs cristallins afin de satisfaire à la condition de diffraction desdits demi-miroirs à rayons X,

un élément à déphasage de rayons X accordable placé dans le trajet d'au moins un des faisceaux de rayons X interférents pour obtenir une image représentant la distribution d'un déphasage de rayons X provoqué par l'objet placé sur un des trajets des faisceaux de rayons X interférents,

lesdites platines d'ajustement, ladite table, lesdits dispositifs de surveillance d'intensité de rayons X, et ledit élément à déphasage de rayons X accordable étant positionnés dans ladite chambre (51),

des capteurs d'image à rayons X pour obtenir des motifs d'interférence de rayons X des faisceaux de rayons X interférents, et

un ordinateur pour commander ledit élément à déphasage de rayons X accordable, lesdites platines d'ajustement, et lesdits capteurs d'image à rayons X, afin d'analyser des motifs d'interférence de rayons X observés pour délivrer des signaux de rétroaction qui sont envoyés aux platines d'ajustement de manière à compenser des dérives de franges d'interférence.

# FIG. 1 PRIOR ART

# FIG. 2(a)  FIG. 2(b)  FIG. 2(c)  FIG. 2(d)

FIG. 3(a)

FIG. 3(b)

FIG. 3(c)

EP 0 799 600 B1

## FIG. 4

## FIG. 5

# FIG. 6

## FIG. 7

<u>200</u>

FIG. 8(a)

FIG. 8(b)

FIG. 8(c)

FIG. 8(d)

FIG. 9

## FIG. 10

## FIG. 11

FIG. 12

FIG. 13

## FIG. 14

FIG. 15

# FIG. 16

# FIG. 17